# EUROPEAN PATENT APPLICATION

(11) **EP 2 783 651 A1**
(43) Date of publication of application: **01.10.2014**
(21) Application number: 12850975.9
(22) Date of filing: 19.11.2012
(51) Int. Cl.: A61B 18/14, A61B 18/00

(54) **RENAL ARTERY ABLATION CATHETER AND SYSTEM**

(30) Priority: 21.11.2011 JP 2011254319
(71) Applicant: Denerve Inc., Chiyoda-ku, Tokyo 1010042 (JP)
(72) Inventor: OKUYAMA, Yuji, Suita-shi Osaka 565-0871 (JP)
(74) Representative: Cabinet Plasseraud
(86) International application number: PCT/JP2012/079916
(87) International publication number: WO 2013/077283

(57) **Abstract**

Provided is a catheter (100) including: a flexible sheath (1); a flexible shaft (2) which is provided by being inserted inside the sheath (1) and which is capable of sliding in a longitudinal direction of the sheath (1); an electrode section (10) which is capable of being in contact with a renal artery wall. The catheter (100) is configured such that a plurality of electrodes (12) provided to the electrode section (10) do not exist on a single plane perpendicular to a moving axis of the shaft (2). According to the present invention, it is possible to easily and quickly perform renal artery ablation while avoiding post-ablation renal artery stenosis.

## Description

### Technical Field

The present invention relates to a catheter and a system for renal artery ablation.

### Background Art

Treatment-resistant hypertension, which is seen in approximately 15% of hypertensive patients, indicates a blood pressure which cannot be controlled even in a case where three types of antihypertensive medicines (including a diuretic) are used in prescribed dosage. In recent years, it has been shown that renal artery ablation can be effective against treatment-resistant hypertension (see Non-Patent Literature 1), and it has been noted that activation of sympathetic nerves plays an important role in cardiovascular diseases such as hypertension and cardiac failure.

The renal artery ablation is a technique of decreasing blood pressure by (i) inserting a catheter having an electrode(s) into a renal artery and (ii) ablating renal nerves surrounding adventitia of the renal artery by radiofrequency electrification at several locations of the renal artery. As being easily and safely performed, the renal artery ablation is greatly expected as a non-pharmacological treatment for hypertension.

### Citation List

Non-Patent Literature 1
Lancet 2009; 373: 1275-1281

### Summary of Invention

### Technical Problem

In a case where the renal artery ablation is performed, ablation points should not be located on an identical cross-section in a short-axis direction of a renal artery for the purpose of avoidance of post-ablation renal artery stenosis. In view of the circumstances, Non-patent Literature 1 discloses that it is necessary to perform ablation while moving an end of a catheter so that the ablation is performed with the ablation points moved as if the ablation points are spirally arranged. This is skilled operation and, accordingly, requires 40 to 50 minutes for one case.

The present invention has been made in view of the above problem, and an object of the present invention is to provide a technique of easily and quickly performing renal artery ablation while avoiding post-ablation renal artery stenosis.

### Solution to Problem

In order to attain the above object, a catheter of the present invention, for renal artery ablation, includes: a flexible sheath; a flexible shaft which is provided by being inserted inside the sheath and which is capable of sliding in a longitudinal direction of the sheath; and an electrode section which is provided at a distal end of the shaft and which is capable of moving between an inside and an outside of the sheath in accordance with the slide of the shaft,
the electrode section including a plurality of elastic wires which are bundled together at their sides proximal to the shaft, each of the plurality of elastic wires being capable of bending to rise, at its side proximal to the shaft, from the longitudinal direction of the sheath, the plurality of elastic wires being close to each other toward a moving axis of the shaft in a case where the electrode section is housed inside the sheath, the plurality of elastic wires spreading in a direction perpendicular to the longitudinal direction of the sheath in a case where the electrode section is pushed outside the sheath, and electrodes for ablation of a renal nerve, the electrodes being provided to the respective plurality of elastic wires in such a way that only one of the electrodes exists on a single plane perpendicular to the moving axis of the shaft.

The catheter of the present invention, for renal artery ablation, includes: a flexible sheath; a flexible shaft which is provided by being inserted inside the sheath and which is capable of sliding in a longitudinal direction of the sheath; and an electrode section which is provided at a distal end of the shaft and which is capable of moving between an inside and an outside of the sheath in accordance with the slide of the shaft,
the electrode section including a plurality of elastic wires which are bundled together at their sides proximal and distal to the shaft, the plurality of elastic wires forming a basket shape by spreading in a direction perpendicular to a moving axis of the shaft in a case where the electrode section is pushed outside the sheath, a region of the basket shape shrinking toward the moving axis in a case where the electrode section is housed inside the sheath, and electrodes for ablation of a renal nerve, the electrodes being provided to the respective plurality of elastic wires forming the basket shape in such a way that only one of the electrodes exists on a single plane perpendicular to the moving axis of the shaft.

The catheter of the present invention is a basket-shaped catheter configured such that a plurality of splines are bundled together and electrodes are provided to the respective plurality of splines in a basket shape at respective specific positions. With this, only by inserting the basket-shaped catheter into a renal artery, it is possible to cause the electrodes to be in close contact with a wall of the renal artery and possible to appropriately arrange ablated areas. Therefore, the catheter is available for a wide range of patients having various diameters of renal arteries. Furthermore, complicated operation of the catheter is not required.

The catheter of the present invention is preferably arranged to further include an ablation checking section for checking whether or not ablation of a renal sensory nerve is successfully performed by the electrodes. With this arrangement, it is possible to easily check whether or not ablation is successfully performed at a target area.

A system of the present invention, for renal artery ablation, includes a catheter recited above and a power source, the power source being electrically connected to electrodes.

### Advantageous Effects of Invention

It is required to have skill in operating conventionally known renal artery ablation catheters. However, a catheter of the present invention allows a user experienced in handling general catheters to easily and suitably perform renal artery ablation. Therefore, the following advantages are expected: (i) a quick and safe catheter-based treatment can be performed on more patients; (ii) a reduction in dosage of an antihypertensive medicine can be achieved; and (iii) a reduction in cardiovascular complications, in distant time from when the renal artery ablation is performed, can be achieved due to an improvement in blood pressure control.

### Brief Description of Drawings

Fig. 1 is a side view schematically illustrating a configuration of a catheter in accordance with the present invention.
Fig. 2 is a side view schematically illustrating a configuration of a catheter in accordance with the present invention.
Fig. 3 is a side view schematically illustrating a configuration of a catheter in accordance with the present invention.
Fig. 4 is a side view schematically illustrating a configuration of a catheter in accordance with the present invention.
Fig. 5 is a cross-sectional view schematically illustrating a configuration of a main part of a catheter in accordance with the present invention.
Fig. 6 is a side view schematically illustrating a configuration of a catheter in accordance with the present invention.

### Description of Embodiments

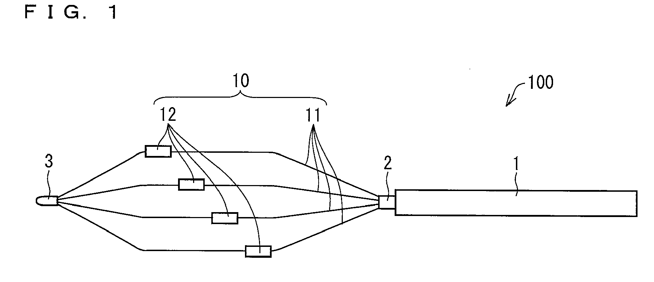

The following description will discuss an embodiment of the present invention with reference to Figs. 1 through 4.

### [1: Embodiment 1]

Fig. 1 is a side view schematically illustrating a configuration of a catheter 100 in accordance with Embodiment 1. The catheter 100 in accordance with Embodiment 1 is configured such that, at its distal end, a spreadable and shrinkable electrode section in a basket shape is mounted (see Fig. 1).

A flexible sheath 1 has, in its inside, an inner cavity (not illustrated) into which a shaft 2 is inserted. The shaft 2, which is capable of moving forward and backward along the inner cavity, is inserted into the inner cavity. An electrode section 10 which is formed in a basket shape from a plurality of elastic wires 11 is connected to an end of the shaft 2. A tapered tip 3 with which ends of the respective plurality of elastic wires 11 bundled together are banded is provided at an end of the electrode section 10. At the other end of the shaft 2, an operation section (not illustrated) of the catheter 100 is provided. For example, biasing means (not illustrated) is connected to the shaft 2. The biasing means causes the shaft 2 to slide. Note that the tip 3 is only necessary to be provided as needed. For example, such an arrangement can be also employed that, without the use of the tip 3, the ends of the respective plurality of elastic wires 11 are bound together in a state where none of the ends of the respective plurality of elastic wires 11 projects.

In a case where the shaft 2 is moved forward and backward in an axis direction of the sheath 1 from a hand side of the operation section with the use of the biasing means, the electrode section 10 moves into and out of the sheath 1. Outside the sheath 1, the electrode section 10 is restored into a basket shape by expanding due to its elasticity. Inside the sheath 1, the electrode section 10 shrinks by being pulled into the sheath 1. Fig. 1 illustrates a state where the electrode section 10 is pushed out of the sheath 1 by the biasing means.

The electrode section 10 includes electrodes 12 provided to the respective plurality of elastic wires 11 bundled together. In a case where the electrode section 10 is pushed out of the sheath 1, the electrode section 10 spreads in a basket shape due to its elasticity, and the electrodes 12 contact with a renal artery wall. With this, it is possible to perform ablation of a renal nerve by radiofrequency electrification. Each of the plurality of elastic wires 11 preferably has a shape-memory property, and can be a shape-memory wire or a shape-memory ribbon which are well-known in this field. This facilitates, for example, self-spread of the electrode section 10 after the electrode section 10 is pushed out of the sheath 1.

After the catheter 100 having such a configuration is inserted into a renal artery of a human subject, the plurality of elastic wires 11 are pushed outside the sheath 1 by pushing the shaft 2. The plurality of elastic wires 11 thus pushed out spreads, so that the electrodes 12 provided to the respective plurality of elastic wires 11 can be in contact with a wall of the renal artery. By electrifying the electrodes 12 in contact with the wall of the renal artery, a renal nerve is ablated.

In a case where a plurality of treatment areas by electrification exist on an identical cross-section in a short axis direction of a renal artery, this increases a risk of forming acute stenosis and/or delayed stenosis in a vessel. According to Embodiment 1, the electrodes 12 are provided to the respective plurality of elastic wires 11 in such a way that the electrodes 12 are not arranged on a single plane perpendicular to a moving axis of the shaft 2. It follows that, in a renal artery, treatment areas by electrification do not exist on an identical cross-section in the short axis direction of the renal artery. It is therefore possible to avoid post-ablation renal artery stenosis.

The drawings illustrate the electrode section 10 having four elastic wires 11. Note, however, the electrode section 10 can have three or five or more elastic wires. On a plane perpendicular to the moving axis of the shaft (that is, plane in a short axis direction of a renal artery), the elastic wires are preferably arranged on a circumference of a single circle whose center is the moving axis of the shaft. Further, central angles formed by adjacent ones of the elastic wires on the circumference are preferably identical. For example, in a case where the number of the elastic wires of the electrode section is three, four, five, and six, the central angles are preferably 120°, 90°, 72°, and 60°, respectively. This facilitates creating such in a renal artery that treatment areas (ablation lesions) by electrification are created at regular intervals at undeviated locations. Obviously, the number of the elastic wires of the electrode section is not limited to such numbers. Further, a degree of each of the central angles is only necessary to be set depending on the number of the elastic wires.

As described above, the electrode section is configured such that the electrodes are arranged in such a way that only one of the electrodes exists on a single plane perpendicular to the moving axis the shaft. The electrodes provided to the respective plurality of elastic wires are preferably arranged so as to spiral toward the moving axis of the shaft. Further, perpendicular lines extending from the respective electrodes to the moving axis of the shaft are preferably at regular intervals along the moving axis of the shaft. This facilitates creating such in a renal artery that treatment areas (ablation lesions) by electrification are created at regular intervals in a long axis direction of the renal artery at undeviated locations.

The sheath 1, the shaft 2, and the plurality of elastic wires 11 of the electrode section 10 are also referred to as an introducer sheath, a guiding catheter, and an ablation catheter, respectively, in this field. The sheath has an outer diameter of 1.0 mm to 8.0 mm, preferably 2.0 mm to 4.0 mm, and has a length of 50 mm to 300 mm, preferably 100 mm to 150 mm. The shaft has an outer diameter of 1.0 mm to 6.0 mm, preferably 1.5 mm to 3.0 mm, and a length of 50 cm to 130 cm, preferably 80 cm to 100 cm. Each of the plurality of elastic wires has an outer diameter of 0.2 mm to 1.5 mm, preferably 0.5 mm to 1.0 mm, and a length of 50 cm to 200 cm, preferably 80 cm to 150 cm.

Materials of the sheath 1, the shaft 2, and each of the plurality of elastic wires 11 are not limited in particular, provided that the materials are ones from which conventional catheters can be made. The sheath 1, shaft 2, and each of the plurality of elastic wires 11 can be made of general soft polyvinyl chloride. Note, however, the sheath 1, shaft 2, and each of the plurality of elastic wires 11 are preferably made of thermoplastic elastomer, more preferably nylon elastomer, styrene elastomer, polyester elastomer, or the like.

A typical example of the nylon elastomer in this field is a block copolymer (for example, polyether block amid copolymer) in which (i) nylon, such as nylon 6, nylon 11, and nylon 12, is a hard segment and (ii) polyether, such as polytetramethylene glycol (PTMG), or a polymer, such as polyester, is a soft segment. Besides, polymer alloy of nylon and flexible resin (polymer blend, a graft polymer, a random polymer, and the like), nylon softened with plasticizer or the like, and mixtures of those can be also preferably used.

As the styrene elastomer, the following can be preferably used: an SBS block copolymer composed of a polystyrene-polybutadiene-polystyrene block; an SIS block copolymer composed of a polystyrene-polyisoprene-polystyrene block; hydrogenated products of those; partially hydrogenated products of those; and mixtures of those.

A typical example of the polyester elastomer is a block copolymer in which (i) saturated polyester, such as polyethylene terephthalate (PET) and polybutylene terephthalate (PBT), is a hard segment and (ii) polyether, such as polytetramethylene glycol (PTMG), or a polymer, such as polyester, is a soft segment. Besides, polymer alloys of those, saturated polyester softened with plasticizer or the like, and mixtures of those can be also preferably used.

Further, surfaces of the sheath 1, the shaft 2, and each of the plurality of elastic wires 11 are each preferably coated by a material (biocompatible material) which can be used in vivo. For example, a polymer compound is preferably used which has a polymer that contains, as a constituent, a monomer such as alkoxyalkyl (meth)acrylate, aminoalkyl (meth)acrylate, aminoalkyl (meth)acrylamide, and quarternary ammonium salt derivatives of those.

For example, the sheath 1 is formed from, for example, a resin tube made of nylon, polyester, and the like. Metallic meshes can be woven into the sheath 1 so that an outer wall of the sheath 1 has an increased torsional rigidity. The sheath 1 is preferably made of an insulating material so that no radiofrequency current is delivered to an unintended area while the electrodes 12 are being electrified. The outer diameter of the sheath 1 is not limited in particular, provided that the sheath 1 is inserted into a renal artery and the electrodes 12 are in contact with a wall of the renal artery. Further, an inner diameter of the sheath 1 is not limited in particular, provided that the shaft 2 and the electrode section 10 are housed inside the sheath 1.

The material of the shaft 2 is also not limited in particular, provided that (i) the material is one from which conventionally known catheters are made and (ii) the material allows a pushing force and a pulling force applied by the biasing means to be transferred to the electrode section 10. Examples of the material include nylon elastomer resin (for example, polyether block amide copolymer). An insulating material may be attached to the shaft 2 so that the shaft 2 has increased strength.

The electrode section 10 may be formed as an individual member other than the shaft 2. Alternatively, the electrode section 10 may be formed by deforming part of the shaft 2. That is, the electrode section 10 may be formed by bending the end of the shaft 2 which is made up of the plurality of elastic wires 11 bundled together. The material of each of the plurality of elastic wires 11 is not limited in particular, provided that the material allows electrification of the electrodes 12. Further, an insulting material may be attached to the plurality of elastic wires 11 so that the plurality of elastic wires 11 has increased strength.

A material of each of the electrodes 12 is not limited in particular, provided that the material allows an ablation lesion to be created in a renal artery wall in a case where the electrodes 12 in contact with the renal artery wall are electrified. Examples of the material include platinum iridium.

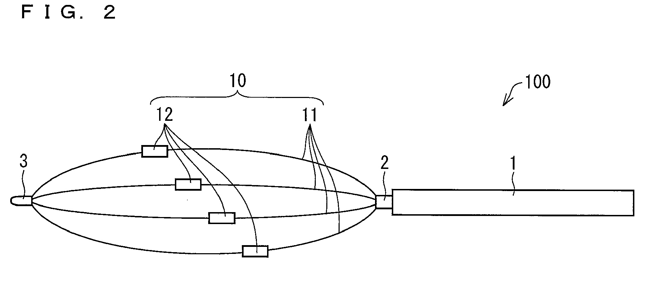

As used in the specification, the term "basket" does not limit a shape to one illustrated in Fig. 1, provided that a plurality of wires are directly or indirectly bundled together at their proximal and distal ends and surround a hollow region. For example, the shape can be an ellipse as illustrated in Fig. 2 or one that is extensible to a sphere or an oval.

### [2: Embodiment 2]

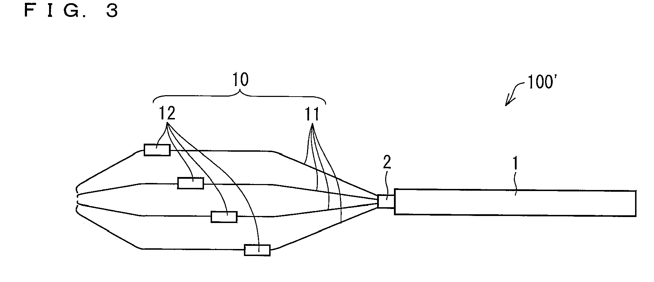

Fig. 3 is a side view schematically illustrating a configuration of a catheter 100' in accordance with Embodiment 2. As illustrated in Fig. 3, the catheter 100' in accordance with Embodiment 2 is configured such that an electrode section 10 is mounted at a distal end of a shaft 2 inserted into a flexible sheath 1, the electrode section 10 being spreadable and shrinkable by moving into and out of the sheath 1 in accordance with slide of the shaft 2. Note that, for convenience, identical reference numbers are given to members having functions identical to those illustrated in Figs. 1 through 2, and no description of the members will be provided here.

According to Embodiment 2, ends of a respective plurality of elastic wires 11 are not bound together (see Fig. 3). That is, each of the plurality of elastic wires 11 connected to the end of the shaft 2 is bent to rise, at its side proximal to the shaft 2, from a longitudinal direction of the sheath 1, and the ends of the respective plurality of elastic wires 11, which ends are situated at an end of the catheter 100', are folded inward. This allows avoidance of damage to an inside of a renal artery into which the catheter 100' is inserted.

In this manner, according to Embodiment 2, the electrode section 10 does not have such a basket shape that both sides of the electrode section 10 are closed as in Embodiment 1, but has such a shape that one of the sides of the electrode section 10 is open. In a case where the electrode section 10 is housed inside the sheath 1, the plurality of elastic wires 11 are close to each other toward a moving axis of the shaft 2. In a case where the electrode section 10 is pushed out of the sheath 1, the electrode section 10 spreads in a direction perpendicular to the longitudinal direction of the sheath 1.

After the catheter 100' having such a configuration is inserted into a renal artery of a human subject, the plurality of elastic wires 11 are pushed outside the sheath 1 by pushing the shaft 2. The plurality of elastic wires 11 thus pushed out spreads, so that electrodes 12 provided to the respective plurality of elastic wires 11 can be in contact with a wall of the renal artery. By electrifying the electrodes 12 in contact with the wall of the renal artery, it is possible to ablate a renal nerve.

Also according to Embodiment 2, the electrodes 12 are provided to the respective plurality of elastic wires 11 in such a way that the electrodes 12 are not arranged on a single plane perpendicular to the moving axis of the shaft 2. It follows that, in a renal artery, treatment areas by electrification do not exist on an identical cross-section in a short axis direction of the renal artery. It is therefore possible to avoid post-ablation renal artery stenosis.

### [3: Other Embodiments]

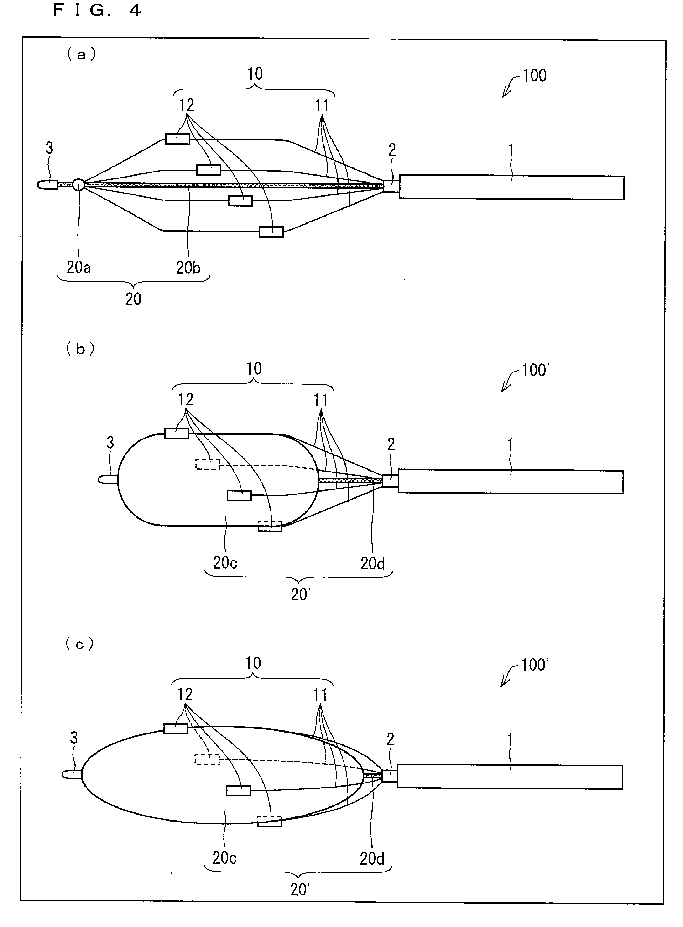

Fig. 4 is a side view schematically illustrating a configuration of a modified example of a catheter in accordance with the present invention. As illustrated in Fig. 4, the catheter in accordance with the present embodiment includes a flexible sheath 1, a shaft 2 inserted into the flexible sheath 1, and a spreadable and shrinkable electrode section 10 mounted at a distal end of the shaft 2. The electrode section 10 includes assisting means 20 which assists a plurality of elastic wires 11 in changing their shapes. Note that, for convenience, identical reference numbers are given to members identical to those illustrated in Figs. 1 through 3, and no description of the members will be provided here.

The assisting means 20 illustrated in (a) of Fig. 4 is a tension wire which is used in combination with the catheter 100 in accordance with Embodiment 1 and which is inserted, together with the shaft 2, into the flexible sheath 1. The assisting means 20 is configured such that it is possible to move, independently of movement of the shaft 2, a bundling section 20a, which bundles the plurality of elastic wires 11 together, along a shaft line 20b, via which an operation section and a tip 3 are connected to each other. Not that, in this case, the tip 3 is provided at an end of the shaft line 20b, but does not bind ends of the respective plurality of elastic wires 11 together.

With this configuration, the electrode section 10 is operationally spread by the assisting means 20. It is therefore possible to better control contact between the electrodes 12 and a vascular wall.

Assisting means 20' illustrated in (b) and (c) of Fig. 4 is constituted by (i) an expansible balloon 20c which expands a plurality of elastic wires 11 by applying an outward force to the plurality of elastic wires 11 and (ii) a tube 20d through which air necessary for the balloon 20c is supplied. The drawings each illustrate a catheter 100' configured such that the balloon 20c, connected to an operation section via the tube 20d, has a tip 3. Note, however, that the catheter 100' can be configured such that the tip 3 is connected to the operation section via the tube 20d and the balloon 20c is connected to the tube 20d so that air is supplied to the balloon 20d through the tube 20d. (b) and (c) of Fig. 4 each illustrate the catheter 100', in accordance with Embodiment 2, combined with the assisting means 20'. According to the catheter 100', the tube 20d is in accordance with movement of a shaft 2. Note, however, that, in a case where the catheter 100 in accordance with Embodiment 1 is combined with the assisting means 20', the catheter 100 can be configured such that it is possible to move the tube 20d, independently of the movement of the shaft 2, in order to move the balloon 20c to a desired position inside the electrode section 10 in a basket shape.

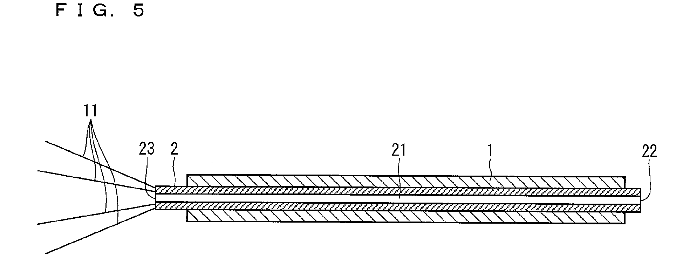

Fig. 5 is a cross-sectional view schematically illustrating a configuration of a main part of a modified example of a catheter in accordance with the present invention. Fig. 5 illustrates a flexible sheath 1, a shaft 2 inserted into the flexible sheath 1, a plurality of elastic wires 11 mounted at a distal end of the shaft 2, and a lumen 21 formed inside the shaft 2. The lumen 21 is formed, inside the sheath 1, in a longitudinal direction of the sheath 1. The lumen 21 has (i) a first opening 22 at its end distal to the plurality of elastic wires 11 and (ii) a second opening 23 at its end proximal to the plurality of elastic wires 11.

The first opening 22 is, directly or via an introduction tube (not illustrated), connected to a medicine containing section (not illustrated) in which a medicine for increasing blood pressure via a renal sensory nerve is contained. The medicine pushed out of the medicine containing section is inserted inside the lumen 21 through the first opening 22, and emitted through the second opening 23 toward the plurality of elastic wires 11 (that is, toward an electrode section 10). By providing a valve between the medicine containing section and the first opening 22, it is possible to control, to a desired timing, a timing at which the medicine is pushed out into the lumen. For example, by (i) using a T shape stopcock for connection between the first opening 22 and syringe serving as the medicine containing section and (ii) opening the T shape stopcock so that the medicine is injected into the lumen 21 through the opening 22, it is possible to successfully control, with a simple configuration, insertion and emission of the mediation into/from the lumen 21. That is, the medicine is preferably contained, in a solution state, in the medicine containing section. The lumen 21 may be filled with a buffer solution (for example, a saline solution) so that the mediation is successfully emitted.

The medicine is not limited to any particular one, provided that the medicine stimulates an ending of a renal sensory nerve, then excitement is transmitted to the central nerve, and blood pressure is consequently increased. Examples of the medicine include adenosine and capsaicin. In a case where adenosine is injected into a renal artery, blood pressure is increased. However, in a case where nerves around the renal artery are denatured, blood pressure is not increased even though adenosine is injected into the renal artery. In view of this, by (i) injecting adenosine into a renal artery before and after ablation by supplying, to the electrode section 10 of the catheter 100, the medicine for increasing blood pressure via a renal sensory nerve and (ii) comparing adenosine-induced blood pressure changes before and after the ablation, it is possible to check whether or not the ablation of the renal sensory nerve is successfully performed.

Fig. 5 illustrates an arrangement such that the first opening 22 is formed at an end of the shaft 2. Note, however, that the first opening 22 can be formed on a side surface of the shaft 2 (in a direction perpendicular to a direction of an axis line of the sheath 1), provided that the first opening 22 does not hinder the sheath 1 from being inserted into a renal artery of a human subject and does not hinder the shaft 2 from sliding. Further, Fig. 5 illustrates an arrangement such that the lumen 21 is formed inside the shaft 2. Note, however, that the lumen 21 can be configured such that the lumen 21 can be inserted, together with the shaft 2, into an inner cavity formed inside the sheath 1, provided that the lumen 21 does not hinder the shaft 2 from sliding.

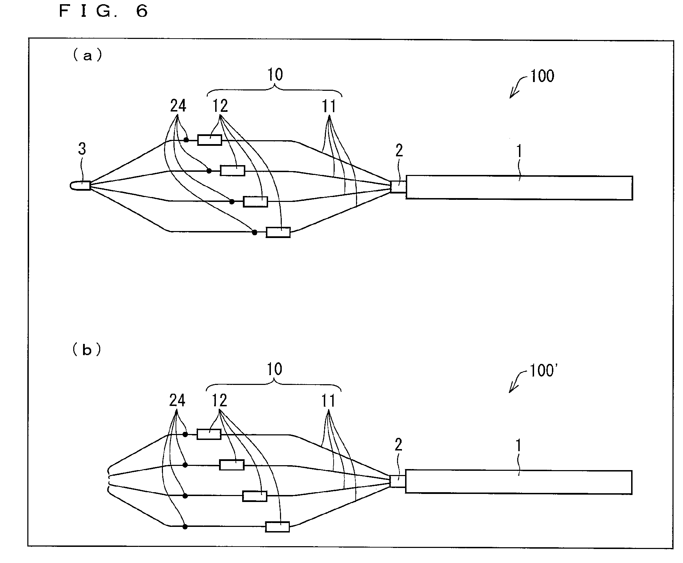

Fig. 6 is a side view schematically illustrating a configuration of a modified example of a catheter in accordance with the present invention. Fig. 6 illustrates catheters 100 and 100' each configured such that an electrode section 10 is mounted at a distal end of a shaft 2, the electrode section 10 being spreadable and shrinkable by moving into and out of a flexible sheath 1 in accordance with slide of a shaft 2 inserted into the sheath 1. According to the catheters 100 and 100', second electrodes 24 are further provided to a respective plurality of elastic wires 11.

By employing a configuration illustrated in each of (a) and (b) of Fig. 6, it is possible to give pulse stimulation at a distal side (peripheral side) of an ablated area while keeping the catheter 100 or 100' in a renal artery. Each of the second electrodes is preferably arranged such that a pair of electrodes (a bipolar electrode) is provided to each of the plurality of elastic wires.

In a case where a renal sensory nerve is cut and pulse stimulation is given to a center side of the renal sensory nerve, the renal sensory nerve is excited, then the excitement is transmitted to the central nerve, and systemic sympathetic activation is consequently increased. As a result, vasopressor reaction is observed. That is, in a case where increased blood pressure, observed before renal artery ablation due to pulse stimulation, is not observed after the ablation even though pulse stimulation is given to a distal side (peripheral side) of an ablated area, it can be said that desired ablation is performed. By thus comparing, before and after ablation, changes in blood pressure due to pulse stimulation to a renal sensory nerve, it is possible to check whether or not the ablation of the renal sensory nerve is successfully performed.

A renal nerve indicates a renal sympathetic efferent nerve and a renal sensory afferent nerve. It is considered that a depressor effect of renal artery ablation for approximately one year after the ablation (so-called acute stage) is brought about because at least one of the renal sympathetic nerve and the renal sensory nerve is ablated. However, it is assumed that, even in a case where the renal sympathetic nerve is damaged, at least part of the renal sympathetic nerve regenerates in distant time from when the ablation is performed. Therefore, it is considered that, because the renal sensory nerve, which is considered not to regenerate, is ablated, blood pressure is kept decreased even three years after the ablation. By providing such an ablation checking section, it is possible to check an effect of ablation in an acute stage, and possible to know an endpoint of a therapeutic effect of the ablation. Furthermore, it is possible to easily and quickly know whether or not intended ablation is successfully performed on a human subject, and possible to know whether or not an effect of renal artery ablation is expected.

As has been described, it is only necessary that a catheter of the present invention include at least: a flexible sheath; a flexible shaft which is provided by being inserted inside the sheath and which is capable of sliding in a longitudinal direction of the sheath; an electrode section which is capable of being in contact with a renal artery wall; and, in the electrode section, electrodes which do not exist on a single plane perpendicular to a moving axis of the shaft. That is, it should be noted that a catheter having a configuration different from those described in the embodiments of the present invention is also encompassed in the technical scope of the present invention.

That is, the object of the present invention is to provide, as a device for renal artery ablation, a catheter including an electrode section which is configured such that a plurality of electrodes do not exist on a single plane perpendicular to a moving axis of a shaft and which is capable of being in contact with a renal artery wall. The present invention does not lie in the material and the shape of each member described in detail in the specification.

Further, the present invention provides a system for renal artery ablation which system includes the foregoing catheter, biasing means, and a power source (not illustrated) electrically connected to electrodes. The system of the present invention can further include a control section which controls output of the power source and/or operation of the biasing means. In this case, a sensor is preferably provided to the catheter which sensor detects a position of the catheter, pressure applied from each of the electrodes to a renal artery wall, or a temperature at a position of each of the electrodes. That is, the system of the present invention preferably includes at least one of a position sensor, a pressure sensor, and a temperature sensor, more preferably a thermistor at each of the electrodes. As the biasing means, a handle made of polycarbonate is preferably employed. However, the biasing means is not limited to such an example.

The system of the present invention can be arranged such that the control section controls insertion and emission of a medicine into/from a lumen by controlling extrusion of the medicine through a valve and from a medicine containing section. Further, the system can be arranged such that the control section controls pulse stimulation by controlling a pulse generating section. In either case, the system of the present invention preferably further includes a blood-pressure measuring section which measures blood pressure of a human subject. In this case, the control section can further control operation of the catheter in accordance with information about blood pressure measured by the blood-pressure measuring section. Note that an area at which blood pressure is measured is not limited to a renal artery. Therefore, the blood-pressure measuring section can be provided at any area, independently of catheter.

As has been described, the present invention can be as follows:
[1] A catheter for renal artery ablation including:
   a flexible sheath;
   a flexible shaft which is provided by being inserted inside the sheath and which is capable of sliding in a longitudinal direction of the sheath; and
   an electrode section which is provided at a distal end of the shaft and which is capable of moving between an inside and an outside of the sheath in accordance with the slide of the shaft,
   the electrode section including a plurality of elastic wires which are bundled together at their sides proximal to the shaft,
   each of the plurality of elastic wires being capable of bending to rise, at its side proximal to the shaft, from the longitudinal direction of the sheath, the plurality of elastic wires being close to each other toward a moving axis of the shaft in a case where the electrode section is housed inside the sheath, the plurality of elastic wires spreading in a direction perpendicular to the longitudinal direction of the sheath in a case where the electrode section is pushed outside the sheath, and
   electrodes for ablation of a renal nerve, the electrodes being provided to the respective plurality of elastic wires in such a way that only one of the electrodes exists on a single plane perpendicular to the moving axis of the shaft.
[2] A catheter as set forth in [1], further including an ablation checking section for checking whether or not ablation is successfully performed by the electrodes.
[3] The catheter as set forth in [2], wherein the ablation checking section is a lumen which is formed inside the sheath or the shaft in the longitudinal direction of the sheath and which has (i) a first opening through which a medicine for increasing blood pressure via a renal sensory nerve is received from the outside of the sheath and (ii) a second opening through which the medicine is emitted toward the electrode section.
[4] A catheter as set forth in [3], further including a medicine containing section in which the medicine is contained.
[5] The catheter as set forth in [3] or [4], wherein the medicine is adenosine or capsaicin.
[6] The catheter as set forth in [2], wherein the ablation checking section is a second electrode for transmitting electric pulses to a renal sensory nerve located on a distal side of an area to be ablated by each of the electrodes.
[7] A catheter as set forth in [6], further including a pulse generating section for generating the electric pulses.
[8] A catheter as set forth in [1] through [7], wherein the plurality of elastic wires are bundled together at their sides which are located on distal sides of the electrodes,
   in a case where the electrode section is pushed outside the sheath, the plurality of elastic wires form a basket shape by spreading in a direction perpendicular to the moving axis of the shaft, and
   in a case where the electrode section is housed inside the sheath, a region of the basket shape shrinks toward the moving axis. That is,
   A catheter for renal artery ablation, including:
   a flexible sheath;
   a flexible shaft which is provided by being inserted inside the sheath and which is capable of sliding in a longitudinal direction of the sheath; and
   an electrode section which is provided at a distal end of the shaft and which is capable of moving between an inside and an outside of the sheath in accordance with the slide of the shaft,
   the electrode section including a plurality of elastic wires which are bundled together at their sides proximal and distal to the shaft,
   the plurality of elastic wires forming a basket shape by spreading in a direction perpendicular to a moving axis of the shaft in a case where the electrode section is pushed outside the sheath,
   a region of the basket shape shrinking toward the moving axis in a case where the electrode section is housed inside the sheath, and
   electrodes for ablation of a renal nerve, the electrodes being provided to the respective plurality of elastic wires forming the basket shape in such a way that only one of the electrodes exists on a single plane perpendicular to the moving axis of the shaft.
[9] The catheter as set forth in [8], wherein each of the plurality of elastic wires forming the basket shape is once or more bent along the moving axis towards its distal end from its portion at which the elastic wire is bent to rise from the longitudinal direction of the sheath.
[10] The catheter as set forth in [1] through [9], wherein the shaft and the plurality of elastic wires bundled together are integrally formed.
[11] A catheter as set forth in [1] through [10], further including biasing means for sliding the shaft.
[12] A catheter as set forth in [1] through [11], further including assisting means for assisting the plurality of elastic wires in changing their shapes in accordance with the movement of the electrode section.
[13] A system for renal artery ablation, including a catheter recited in [1] through [12] and a power source, the power source being electrically connected to electrodes.
[14] A system as set forth in [13], further including (i) at least one of a position sensor, a pressure sensor, and a temperature sensor and (ii) a control section for controlling, in accordance with in vivo information obtained by the least one of the position sensor, the pressure sensor, and the temperature sensor, output from the power source and/or operation of biasing means.
[15] A system as set forth in [13] or [14], further including a blood-pressure measuring section.
[16] A method for performing renal artery ablation, including the steps of:
   (a) inserting a catheter into a renal artery of a human subject, the catheter including: a flexible sheath; a flexible shaft which is provided by being inserted inside the sheath and which is capable of sliding in a longitudinal direction of the sheath; and an electrode section which is provided at a distal end of the shaft and which is housed inside the sheath, the electrode section including a plurality of elastic wires which are bundled together at their sides proximal to the shaft and electrodes for ablation of a renal nerve, the electrodes being provided to the respective plurality of elastic wires in such a way that only one of the electrodes exists on a single plane perpendicular to the moving axis of the shaft;
   (b) pushing the shaft so that the electrode section is pushed outside the sheath;
   (c) causing the electrodes to be in contact with a wall of the renal artery by further pushing the shaft so that the plurality of elastic wires spread; and
   (d) ablating a renal nerve by electrifying the electrodes in contact with the wall of the renal artery.
[17] A method as set forth in [16], further including the step of (e) checking whether or not ablation of a renal sensory nerve is successfully performed.
[18] The method as set forth in [17], wherein the step (e) is carried out by supplying, to the electrode section, a medicine for increasing blood pressure via a renal sensory nerve.
[19] The method as set forth in [18], wherein the medicine is supplied to the electrode section in such a manner that the medicine is (i) injected inside the sheath from an outside of the sheath through a first opening formed in a lumen which is formed inside the sheath or the shaft in the longitudinal direction of the sheath and (ii) emitted toward the electrode section.
[20] The method as set forth in [18] and [19], wherein the medicine is adenosine or capsaicin.
[21] The method as set forth in [17], wherein the step (e) is carried out by transmitting electric pulses to the renal sensory nerve located on a distal side of an area to be ablated by each of the electrodes.
[22] The method as set forth in [21], wherein the electric pulses are transmitted from a pulse generating section provided outside the sheath to a second electrode provided to the electrode section.
[23] A method as set forth in [16] through [22], further including the step of (f) measuring blood pressure of the human subject.
[24] A method as set forth in [16] through [23], further including the step of (g) taking out the catheter from the renal artery of the human subject after housing the electrode section inside the sheath by pulling the shaft.

The present invention is not limited to the description of the embodiments, but may be altered by a skilled person in the art within the scope of the claims. An embodiment derived from a proper combination of technical means disclosed in different embodiments is also encompassed in the technical scope of the present invention.

All of the academic literatures and the patent literatures stated in the specification are incorporated in the specification by way of reference.

### Industrial Applicability

It is expected that the present invention is applicable to hypertensive patients or the other disease conditions based on excess activation of sympathetic nerves, especially to treatment-resistant hypertension.

### Reference Signs List

- 1: Sheath
- 2: Shaft
- 10: Electrode section
- 11: Elastic wires
- 12: Electrodes
- 100: Catheter

## Claims

1. A catheter for renal artery ablation, comprising:
a flexible sheath;
a flexible shaft which is provided by being inserted inside the sheath and which is capable of sliding in a longitudinal direction of the sheath; and
an electrode section which is provided at a distal end of the shaft and which is capable of moving between an inside and an outside of the sheath in accordance with the slide of the shaft,
the electrode section including a plurality of elastic wires which are bundled together at their sides proximal to the shaft,
each of the plurality of elastic wires being capable of bending to rise, at its side proximal to the shaft, from the longitudinal direction of the sheath, the plurality of elastic wires being close to each other toward a moving axis of the shaft in a case where the electrode section is housed inside the sheath, the plurality of elastic wires spreading in a direction perpendicular to the longitudinal direction of the sheath in a case where the electrode section is pushed outside the sheath, and
electrodes for ablation of a renal nerve, the electrodes being provided to the respective plurality of elastic wires in such a way that only one of the electrodes exists on a single plane perpendicular to the moving axis of the shaft.

2. A catheter as set forth in claim 1, further comprising an ablation checking section for checking whether or not ablation is successfully performed by the electrodes.

3. The catheter as set forth in claim 2, wherein the ablation checking section is a lumen which is formed inside the sheath or the shaft in the longitudinal direction of the sheath and which has (i) a first opening through which a medicine for increasing blood pressure via a renal sensory nerve is received from the outside of the sheath and (ii) a second opening through which the medicine is emitted toward the electrode section.

4. A catheter as set forth in claim 3, further comprising a medicine containing section in which the medicine is contained.

5. The catheter as set forth in claim 3 or 4, wherein the medicine is adenosine or capsaicin.

6. The catheter as set forth in claim 2, wherein the ablation checking section is a second electrode for transmitting electric pulses to a renal sensory nerve located on a distal side of an area to be ablated by each of the electrodes.

7. A catheter as set forth in claim 6, further comprising a pulse generating section for generating the electric pulses.

8. The catheter as set forth in any one of claims 1 through 7, wherein:
the plurality of elastic wires are bundled together at their sides which are located on distal sides of the electrodes,
in a case where the electrode section is pushed outside the sheath, the plurality of elastic wires form a basket shape by spreading in a direction perpendicular to the moving axis of the shaft, and
in a case where the electrode section is housed inside the sheath, a region of the basket shape shrinks toward the moving axis.

9. The catheter as set forth in claim 8, wherein each of the plurality of elastic wires forming the basket shape is once or more bent along the moving axis towards its distal end from its portion at which the elastic wire is bent to rise from the longitudinal direction of the sheath.

10. The catheter as set forth in any one of claims 1 through 9, wherein the shaft and the plurality of elastic wires bundled together are integrally formed.

11. A catheter as set forth in any one of claims 1 through 10, further comprising biasing means for sliding the shaft.

12. A catheter as set forth in any one of claims 1 through 11, further comprising assisting means for assisting the plurality of elastic wires in changing their shapes in accordance with the movement of the electrode section.

13. A system for renal artery ablation, comprising a catheter recited in any one of claims 1 through 12 and a power source, the power source being electrically connected to electrodes.

14. A system as set forth in claim 13, further comprising (i) at least one of a position sensor, a pressure sensor, and a temperature sensor and (ii) a control section for controlling, in accordance with in vivo information obtained by the least one of the position sensor, the pressure sensor, and the temperature sensor, output from the power source and/or operation of biasing means.

15. A system as set forth in claim 13 or 14, further comprising a blood-pressure measuring section.
